# EUROPÄISCHE PATENTSCHRIFT

(11) **EP 2 983 733 B1**
(45) Veröffentlichungstag und Bekanntmachung des Hinweises auf die Patenterteilung: **02.12.2020**
(21) Anmeldenummer: 14713455.5
(22) Anmeldetag: 27.03.2014
(51) Int. Cl.: A61M 1/16, B01D 19/00, C02F 1/20

(54) **METHODE ZUR ULTRASCHALL-ENTGASUNG VON FLÜSSIGKEITEN FÜR DIE DIALYSE**
METHOD FOR THE ULTRASONIC DEGASSING OF LIQUIDS FOR DIALYSIS
PROCÉDÉ DE DÉGAZAGE PAR ULTRASONS DE LIQUIDES DE DIALYSE

(30) Priorität: 11.04.2013 DE 102013006667
(43) Veröffentlichungstag der Anmeldung: 17.02.2016
(73) Patentinhaber: Fresenius Medical Care Deutschland GmbH, 61352 Bad Homburg (DE)
(72) Erfinder: KOCH, Michael, 97447 Gerolzhofen (DE)
(74) Vertreter: Breuninger, Marcus
(86) Internationale Anmeldenummer: PCT/EP2014/056155
(87) Internationale Veröffentlichungsnummer: WO 2014/166748

(56) Entgegenhaltungen:
- EP-A1- 1 820 550
- WO-A1-01/41655
- WO-A1-97/20612
- US-A- 5 022 899
- US-A1- 2007 045 188
- US-A1- 2009 084 718

## Beschreibung

### Technisches Gebiet

Gegenstand der Erfindung ist eine Vorrichtung und ein Verfahren für die Ultraschallentgasung von Flüssigkeiten, die in der Dialyse Verwendung finden.

In der Dialyse wird das Blut nierengeschädigter Patienten mit einer Austauschflüssigkeit über eine Dialysemembran in Kontakt gebracht. Solche Dialysierflüssigkeiten nehmen toxische Stoffwechselprodukte aus dem Blut auf, die durch den Patienten selbst nicht mehr ausgeschieden werden können und werden verworfen, so dass sich durch die Dialyse ein Reinigungseffekt des behandelten Blutes einstellt.

In der Hämodialyse findet der Stoffaustausch an einer künstlichen Membran in einem extrakorporalen Blutkreislauf statt, wohingegen in der Peritonealdialyse das Bauchfell des Patientenperitoneums als Austauschmembran genutzt wird.

Darüber hinaus werden Behandlungsflüssigkeiten in der Therapie der Dialyse und anderen Therapien von extrakorporalen Blutbehandlung auch direkt in das zu behandelnde Blut infundiert.

Aus medizinischer Sicht ist es dabei unzulässig und unter Lebensgefahr für den Patienten absolut zu vermeiden, dass in derartigen Infusionsschritten mit der Behandlungsflüssigkeit auch eventuell vorkommende Gaseinschlüsse infundiert werden.

Auch in Behandlungen der Dialyse, bei denen die Behandlungsflüssigkeit durch die Membran getrennt mit dem Blut in Stoffaustauschkontakt steht, können Gaseinschlüsse in der Behandlungsflüssigkeit eine medizinisch gefährlich Situation für den Patienten hervorrufen.

Dialysemaschinen, mit deren Hilfe die extrakorporale Blutdialyse oder die Peritonealdialyse durchgeführt werden, sind durch ein komplexes System von Regelkreisläufen, Mess- Steuer und Alarmeinrichtungen gekennzeichnet. Eventuelle Gaseinschlüsse in Behandlungsflüssigkeiten werden fortwährend durch ein entsprechendes Überwachungssystem detektiert und durch Alarmsignale angezeigt. Andernfalls würden Druckmesseinrichtungen oder Bilanziereinrichtungen solcher Dialysemaschinen fehlerhafte Ausgaben erstellten, so dass eine Behandlung des Patienten gestört würde oder nicht durchführbar wäre.

In Abschnitten, in denen die Dialyierflüssigkeit durch die Förderpumpeneinrichtungen einem Unterdruck ausgesetzt sind, kann bei zu hoher Gasbefrachtung der Behandlungsflüssigkeit eine spontane Gasabscheidung in Form von eingeschlossenen Gasblasen beobachtet werden. Solche Situationen treten im Dialysierflüssigkeitszweig des extrakorporalen Blutkreislaufs besonders stromabwärts von Drosselungsstellen auf. Solche Drosselungsstellen sind z.B. Verengungen, hinter denen ein signifikanter Druckabfall eintritt, insbesondere dann, wenn die Förderpumpe ebenfalls stromabwärts der Drosselungsstelle liegt.

Bei Dialyseverfahren der extrakorporalen Blutbehandlung stellt bereits der Dialysefilter eine solche Drosselungsstelle da. Es besteht also insgesamt immer die Notwendigkeit, wenn die Behandlungsflüssigkeiten mit dem zu behandelnden Blut in Kontakt kommt, dass die Behandlungsflüssigkeiten sorgfältig entgast werden.

Weiterhin stellt die durch das thermodynamische Gas-Lösungsgleichgewicht hervorgerufene Entgasung einer Behandlungsflüssigkeit bei Temperaturerhöhung eine Quelle für das Auftreten von Gaseinschlüssen dar. Bei erhöhten Temperaturen ist die Lösungskapazität von Gasen in einer wässrigen Lösung erniedrigt. Eine Behandlungsflüssigkeit, die bei Raumtemperatur hergestellt wird und anschließend auf die physiologische Temperatur des Patienten erwärmt wird, tendiert zwangsläufig dazu, gelöste Gase gasförmig abzuscheiden.

Darüber hinaus kann eine Gasbefrachtung aber auch durch Mikroblasen vorliegen, die sich nur langsam aus der Behandlungsflüssigkeit abtrennen. Insbesondere kann eine solche Befrachtung auftreten, wenn die Behandlungsflüssigkeiten aus sirupösen, pastösen oder trockenen Konzentraten durch Verdünnung und Verwirbelung mit Wasser hergestellt werden. Die Konzentrate selbst können schon mikroskopische Gaseinschlüsse aufweisen, die bei Verdünnung mit Wasser in die Gaseinschlüsse aufweisen, die bei Verdünnung mit Wasser in die Behandlungsflüssigkeit aufgenommen werden.

### Stand der Technik

Es ergibt sich daher für Dialysemaschinen stets die Notwendigkeit, Behandlungsflüssigkeiten zu entgasen. Im Stand der Technik wird das Entgasen von Behandlungsflüssigkeiten in Dialysemaschinen beschrieben:

Die DE 40 20 840 A1 beschreibt eine Vorrichtung zum Entgasen einer Flüssigkeit durch Hitzeanwendung. Die zu entgasende Flüssigkeit wird dabei bis nahe an den Siedepunkt erhitzt und an einem Hohlfasermembranmodul, der als Wärmetauscher dient, wieder abgekühlt.

JP 2002018202 beschreibt eine Entgasungsvorrichtung für tragbare Dialysegeräte. In einer Kammer wird durch Ultraschallanwendung eine Separation von Flüssigkeit und Gaseinschlüssen bewirkt. Die abgetrennten Gaseinschlüsse können über Rinnen der Kammerwandung ausgeleitet werden.

Die US 2009/0084718 offenbart ein Dialysekassette, die eine Luftabscheidekammer aufweist. Die in die Luftabscheidekammer geleitete Flüssigkeit kann auf verschiedene Art und Weise entgast werden. Beispielsweise wird vorgeschlagen, die Flüssigkeit gegen Hindernisse, wie beispielsweise eine am Boden der Kammer befestigte Prall- / oder Ablenkfläche oder gegen eine als kleine Kugeln oder eine Helix geformte Hindernisfläche zu leiten, so dass die Flussgeschwindigkeit verringert wird. Alternativ wird vorgeschlagen, die Luftabscheidekammer in Kontakt mit einer Schwingungen erzeugenden Vorrichtung zu bringen, um Lufteinschlüsse freizusetzen .

Die WO 97/20612 beschreibt eine Vorrichtung zur Entfernung von Gasblasen, bestehend aus einem Behältnis mit einem Auslass und einem Einlass, die über die gesamte Länge des Behältnisses voneinander beabstandet sind und Mitteln zur Übertragung eines Ultraschallsignals aus einem Signalgeber außerhalb des Behältnisbodens über ein signalweiterleitendes Element (20) in Richtung des Einlasses.

Im Stand der Technik ebenfalls bekannt ist eine Entgasung durch Anwendung von Unterdruck. Dabei wird in einer Kammer einer Entgasungseinheit die zu entgasende Flüssigkeit einem Unterdruck ausgesetzt. Die Flüssigkeit wird durch eine Pumpe ausgeleitet und in einer zweiten Kammer von den abgeschiedenen Gaseinschlüssen getrennt. Die Flüssigkeit muss durch eine Pumpenmechanik aus der Entgasungseinheit herausgeführt werden. Beim Durchlaufen der Pumpenmechanik wird ein Teil des abgetrennten Gases wieder in der Flüssigkeit gelöst. Nach diesem Prinzip kann eine Entgasung nie so vollständig verlaufen, wie es die Vakuumanwendung an sich zuließe.

Das Prinzip des Rücklösens von Gas in die Flüssigkeit in Entgasungseinheiten von Dialysemaschinen ist ein generelles Problem, die die Entgasung ineffizient macht. Dieser Nachteil schlägt sich zum Teil im apparativen Aufbau der Entgasungeinheiten nieder. Die Entgasungeinheit muss entsprechend größer ausgelegt werden, damit die in Kauf zu nehmenden Rücklösungserscheinungen durch eine größere Entgasungsleistung der Anlage kompensiert werden können. Eine Effizienzsteigerung erzielt damit nicht nur eine Energieersparnis im Betrieb der Entgasungseinheit. Die gesamte Entgasungseinheit kann dann auch kleiner ausgelegt werden und die Apparatur wird kostengünstiger.

Gegenwärtige Entwicklungen von Dialysemaschine gehen zunehmend in die Richtung, die Bestandteile des extrakorporalen Blutkreislaufs aus Einmalartikeln aufzubauen. Man erhofft sich durch diese Entwicklung eine schnellere Handhabung für das Klinikpersonal, insbesondere indem Aufrüst- und Abrüstzeiten an den Dialysemaschinen verringert werden.

In Folge gehen die Entwicklungen dahin, dass die Einmalartikel hoch integrierte Systeme darstellen, die immer mehr Funktionalitäten beinhalten. Im günstigsten Fall ist nach diesen Bestrebungen der Einmalartikel so konstruiert, dass er sämtliche notwendigen Funktionalitäten für die Dialyse bereits beinhaltet.

Es kann so auf Desinfektions- und Spülvorgänge der Behandlungsvorrichtung weitgehend verzichtet werden. Die Behandlungseinrichtung wird dadurch kostengünstiger und universeller einsetzbar.

Den konstruktiven technischen Möglichkeiten der Einmalartikeln sind natürlich Grenzen gesetzt. Bauliche Größe, Gewicht und Material eines Einmalartikels sind in Hinblick auf Handhabbarkeit, Preis und Entsorgung limitiert.

Es besteht somit gegenüber dem Stand der Technik das Bedürfnis, eine Entgasungseinheit für Flüssigkeiten, die in der Nierendialyse oder in der extrakorporalen Blutbehandlung relevant sind, bereitzustellen, die einen nur geringen apparativen Aufbau beinhaltet und damit kostengünstig im Betrieb und der Herstellung ist.

### Darstellung der Erfindung

Eine Aufgabe der vorliegenden Erfindung ist es daher, eine Entgasungsapparatur zu finden, die auf die gängigen Entgasungskomponenten von Vakuumanwendung und Hitzeanwendung verzichtet und auf einem Einmalartikel untergebracht werden kann.

Eine weitere Aufgabe bestand darin, die Entgasungstechnologie so effizient wie möglich zu gestalten, so dass trotz entsprechender Miniaturisierung eine gute Entgasungsleistung erzielt werden kann.

Die Aufgabe wird durch die Ansprüche 1, 10, 11 und 12 gelöst. Die Merkmale der Unteransprüche geben bevorzugte Ausführungsformen der Erfindung wieder.

Demnach umfasst eine Vorrichtung zum Entgasen einer Behandlungsflüssigkeit, die auch als Entgasungseinheit bezeichnet werden kann, ein Behältnis, das die zu entgasende Behandlungsflüssigkeit für die Dialyse aufnimmt. Darin angeordnet ist eine Ultraschall-Sonotrode, die dafür vorgesehen ist, die Ultraschall Energie auf die Flüssigkeit zu übertragen. Im vorliegenden Zusammenhang soll als Sonotrode die Kombination aus einem Schwinger und einem Resonator verstanden werden. Der Schwinnger erzeugt oder überträgt Schwingungen, die als Ultraschallsignal in die zu entgasende Flüssigkeit eingeleitet wird. Der Resonator ist an den Schwinger so gekoppelt, dass er die Schwingungen des Schwingers auf die Flüssigkeit überträgt.

Der Resonator wird dabei von der Flüssigkeit umspült, oder taucht in die Flüssigkeit, die sich in dem Behältnis befindet, ein. Durch Aktivierung des Resonators wird eine Entgasung der Behandlungsflüssigkeit bewirkt. Der Resonator zeichnet sich dabei weiterhin dadurch aus, dass er einen inneren Hohlkanal beinhaltet, über den das durch den Ultraschall separierte Flüssigkeits-/ Gasgemisch aus dem Behältnis ausgeleitet werden kann.

Durch diese Anordnung wird der erzeugte Ultraschall über den Resonator direkt in die Flüssigkeit eingeleitet. In direkter Nähe des Resonators ist die Entgasungsleistung am größten. Über den eingearbeiteten Hohlkanal des Resonators kann das entgaste Gemisch aus Flüssigkeit und getrenntem Gas direkt aus der Entgasungseinheit ausgeleitet werden, so dass Rückmischungen des Gases vermieden werden können. Letztendlich wird durch simultane erzeugte Entgasung und Absaugung ein Effektivitäts-/Effizienzoptimum erreicht, das es erlaubt die Bauteilgröße gering zu halten. Die geringe Bauteilgröße ermöglicht es, die Entgasungseinheit auf einem Einwegartikel, auch als Disposable bezeichnet, für Dialysebehandlungen oder der extrakorporalen Blutbehandlung unterzubringen.

Bei dem Behältnis kann es sich um eine für die Aufnahme von Flüssigkeiten vorbereitete Kammer in einer Dialysemaschine oder auf einem Einmalartikel zur Dialysebehandlung handeln. Wenn in einer Ausführung die Entgasung kontinuierlich an durchströmender Flüssigkeit stattfindet, kann die Kammer ein Volumen von 1 ml bis 500 ml umfassen. Alternativ können auch Volumina von 5 ml bis 350 ml oder 5 ml bis 250 ml für die Entgasungseinheit vorgesehen sein.

In Fällen, in denen die Entgasungseinheit ein Teil eines Einwegartikels, z.B. einem integrierten Kassettenmodul für die extrakorporale Blutbehandlung oder die Peritonealdialyse darstellt, sind für die Entgasungseinheit entsprechend kleinere Volumina vorzusehen. In diesem Fall können Kammern von 1 ml bis 50 ml in Frage kommen.

Die Größe der Kammern richtet sich auch nach dem Strömungsprinzip, mit der die zu entgasenden Flüssigkeiten durch den Einmalartikel geleitet werden. Bei diskontinuierlicher Strömung sind entsprechend größere Volumina für die Kammer vorzusehen, wohingegen kleinere Kammervolumina bei kontinuierlichem Strömungsprinzip Anwendung finden.

Darüber hinaus ist die Leistung des Ultraschallschwingers von entscheidender Bedeutung, mit welcher Strömungsrate und gegebenenfalls mit welchem Strömungsprinzip die zu entgasende Flüssigkeit durch die Entgasungseinheit transportiert werden kann. Es haben sich für die in Frage kommenden Anwendungen der Entgasung von Flüssigkeiten für die Dialyse Leistungsabgaben des Schwingers von 50 W bis 1000 W als vorteilhaft erwiesen.

Ultraschall-Sonotroden zur Behandlung von Flüssigkeiten sind im Allgemeinen bekannt. Die Sonotroden besitzen eine stabförmige oder spitzenförmige Geometrie, womit hierunter verstanden wird, dass der Querschnittsdurchmesser der Sonotrode geringer ist als die längliche Ausdehnung.

In der erfindungsgemäßen Ausgestaltung des Resonators, der als Spitze ausgearbeitet ist, ergibt sich der Vorteil, dass der größte Leistungseintrag der Resonatorschwingung in die zu entgasende Flüssigkeit an der Spitze des Resonators erfolgt. Durch den in dem Resonator innen liegenden Ableitkanal kann ein Gemisch von entgaster Flüssigkeit und getrenntem Gas gemeinsam ausgeleitet werden. Da das Gemisch nicht über eine eigene Pumpe ausgeleitet werden muss, besteht nicht die Gefahr, dass Rückmischungen des abgetrennten Gases mit der Flüssigkeit in einer ausleitenden Pumpenmechanik stattfinden.

Gemäß dem Prinzip von Schwinger und Resonator breiten sich die vom Schwinger erzeugten mechanischen Wellen longitudinal in Erstreckungsrichtung des Resonators aus. An der Spitze des Resonators wird die mechanische Aktuierung auf die umgebende Flüssigkeit übertragen. Sofern die Sonotrodenspitze konisch oder hyperboloid zuläuft, findet zusätzlich eine Erhöhung der mechanischen Energiedichte an der Spitze statt. In einer Ausführung, in der der Schwinger als längliche Spitze ausgearbeitet ist, wird daher der Leistungseintrag in die zu entgasende Flüssigkeit bei gegebener Frequenz und Leistung der Sonotrode am größten.

Ein Resonator ist gewöhnlich an einen Schwinger gekoppelt, der piezo-elektrisch erzeugte mechanische Schwingungen erzeugt oder solche Schwingung von einem separaten Schwingungsgenerator überträgt. Ein Piezo-Element wird dabei durch Anwendung einer elektrischen Spannung gemäß dem piezo-elektrischen Effekt in Schwingung versetzt. In der Regel werden Schwingungsfrequenzen oberhalb 20.000 Herz (20 kHz) verwendet, damit sie für das menschliche Ohr nicht wahrnehmbar sind. Für die vorliegende Erfindung wird ein Resonator mit Frequenzen oberhalb 20 kHz betrieben, insbesondere sind Frequenzen von 25 bis 40 kHz vorteilhaft.

Schwinger und Resonator können innerhalb des Behältnisses, das auch als Entgasungseinheit zu bezeichnen ist, auf einem Einmalartikel integriert sein. Solche Einmalartikel können aus einem biegesteifen Spritzgussmaterial, z. B. Polypropylen, hergestellt sein. Die Entgasungseinheit kann einen Teil auf dem Einmalartikel einnehmen. Die Entgasungseinheit kann auf dem Einmalartikel über elektrische Kontaktstellen verfügen, die während der Dialysebehandlung mit einer Dialysemaschine verbunden sind und die notwendige elektrische Energie für die Sonotrode übermitteln.

Unter einer Behandlungsflüssigkeit werden im Zusammenhang dieser Erfindung alle Flüssigkeiten verstanden, die für die Behandlung des dialysepflichtigen Patienten während einer Behandlung notwendig sind. Insbesondere sind darunter zu verstehen Dialysierflüssigkeiten der Hämodialyse und Peritonealdialyse. Darüber hinaus auch Substitutions- und Infusionsflüssigkeiten. Weiterhin auch Spülflüssigkeiten mit denen die Einwegartikel vor der Behandlung vorgespült und luftfrei gefüllt werden.

Unter Dialyse werden alle Behandlungsverfahren verstanden, die mit einer Blutreinigung einhergehen und bei denen die Blutreinigung über eine Membran stattfindet. Beispielhaft, aber nicht erschöpfend werden genannt: Dialyseverfahren, Hämofiltrationsverfahren, Hämodiafiltrationsverfahren, Plasmaphereseverfahren und Peritonealdialyseverfahren.

Im Allgemeinen können bei der Ultraschallbehandlung von Flüssigkeiten Vakuolen im mikroskopischen Maßstab entstehen. Beim Zusammenfallen dieser Vakuolen werden im hörbaren Bereich Schallwellen ausgesendet, die für das Umfeld störend als Kavitationsgeräusche wahrgenommen werden. Insbesondere im therapeutischen Bereich sind solche Geräuschentwicklungen zu vermeiden, oder schalldämmend abzuschirmen. Es hat sich erwiesen, dass durch eine hyperbolische Form des Schwingers unangenehme Kavitationsgeräusche vermieden werden können. In einer Ausführung ist daher vorgesehen, dass der Schwinger ähnlich einem Hyperboloid ausgestaltet ist. Die Seitenflächen zeigen dabei im Längsquerschnitt eine hyperbolische Kontur auf.

Ein weiterer Vorteil der Ultraschallentgasung liegt darin, dass an offenen Entgasungseinheiten gearbeitet werden kann. Darunter ist zu verstehen, dass die Entgasungseinheit im Druckausgleich mit anderen Flüssigkeitsführenden Abschnitten stehen kann. Entgasungseinheiten der Vakuumentgasung benötigen z.B. geschlossene Kammern, so dass nur die Möglichkeit besteht das Flüssigkeits- /Gasgemisch über Pumpen aus der Entgasungseinheit abzuleiten. Nachteilig wirkt sich dabei aus, dass das Gas im Flüssigkeits-/Gasgemisch innerhalb der Pumpenmechanik teilweise in der Flüssigkeit zurückgelöst wird, was den Entgasungsprozess ineffizient macht.

In der vorliegenden Erfindung kann das behandelte Flüssigkeits-/Gasgemisch aus der Entgasungseinheit ausgeleitet werden, indem in der Entgasungseinheit ein leichter Überdruck angelegt wird. Der Überdruck kann sich durch die zuströmende Flüssigkeit im kontinuierlichen Strömungsprinzip aufbauen. Alternativ kann durch Pumpenmittel ein leichter Gasüberdruck im Gasraum über der Flüssigkeit eingestellt werden.

Die Entgasungseinheit ist in diesem Fall mit einem Zulaufkanal verbunden, durch den zuströmende Flüssigkeit in die Entgasungseinheit gefördert wird. Bei den Pumpenmittel kann es sich z. B. um Membranpumpen handeln. Alternativ kann der Überdruck auch durch ein Druckreservoir bereitgestellt werden. Weiterhin verfügt die Entgasungseinheit über einen Anschluss, über den der Entgasungseinheit ein Gas zugeleitet werden kann. Darüber hinaus erfolgt die Ableitung des Flüssigkeits-/Gasgemisches wie beschrieben über den Hohlkanal im Resonator.

Es ist ausreichend, wenn der Überdruck durch einen Druck von wenigen mbar über Atmosphärendruck eingestellt wird. Der Überdruck kann je nach Betriebszustand der Entgasungseinheit 5 bis 200 mbar, bis zu 50 mbar oder bis zu 100 mbar betragen.

Es ist auch vorgesehen, dass das Entgasungsgemisch, das über den Hohlkanal des Schwingers aus der Entgasungseinheit abgeleitet wird in ein zweites Behältnis geleitet wird, das als Abscheidekammer bezeichnet werden kann. Abscheidekammern zum Abscheiden von Gasen und Flüssigkeiten sind im Stand der Technik bekannt. Bevorzugt sind solche Abscheidkammern, in denen das Flüssigkeits-/ Gasgemisch tangential in eine Abscheidekammer mit rundem Querschnitt eingeleitet wird.

Dabei ist es nicht notwendig, dass Resonator und Schwinger fest mit der Entgasungseinheit verbunden sind. Vorteilhaft kann eine Ausgestaltung sein, in der Resonator und Schwinger als Schwimmer ausgebildet sind. Der Schwinger wird als Schwimmer immer auf der Flüssigkeitsoberfläche aufliegen und auch bei veränderlichen Flüssigkeitspegeln in die Flüssigkeit eintauchen. Alternativ kann vorgesehen sein, dass der Schwimmer über eine Führung mit der Entgasungseinheit verbunden ist, aber innerhalb der Führung frei mit dem veränderlichen Flüssigkeitshorizont beweglich ist. Es ergibt sich dadurch der Vorteil, dass der Schwimmer immer in einer optimalen Eintauchtiefe arbeiten und die Entgasung unabhängig vom Flüssigkeitsspiegel effektiv verlaufen kann.

In einer alternativen Ausführung sind Entgasungseinheit und Resonator so ausgestaltet, dass der Resonator vollständig in die Flüssigkeit eintaucht. Insbesondere ist vorgesehen, dass der hyperbolische Körper des Resonators so angeordnet ist, dass die Spitze in der Nähe der Flüssigkeit/Luft oder Flüssigkeit/Gas-Grenzfläche liegt und der gesamte Resonator innerhalb der Flüssigkeit liegt. Der Schwinger braucht dabei nicht oder nicht vollständig von der Flüssigkeit umgeben zu sein. Wichtig ist in diesem Zusammenhang nur, dass der Resonator im größtmöglichen Kontakt mit der zu entgasenden Flüssigkeit steht. Die Anordnung von Resonator, Entgasungseinheit und Schwinger kann dabei so aussehen, dass der Schwinger als ein Teil in der Wandung der Entgasungseinheit eingebaut ist. Über Dichtungen wird eine flüssigkeits- und gasdichte Verbindung zwischen der Wandung der Entgasungseinheit und dem Schwinger hergestellt. Dichtungsmittel hierzu sind dem Fachmann hinlänglich bekannt.

Vorteilhaft wird das entgaste Gemisch durch den Hohlkanal des Resonators und teilweise gebildete Gasblasen an der Spitze in entgegengesetzte Richtungen weggeführt. Die Gasblasen steigen entsprechend dem Auftrieb nach oben zur Flüssigkeits-/Gas-Grenzfläche. Das entgaste Gemisch wird entsprechend der Geometrie des Resonators nach unten von der Grenzfläche weggeführt. Dies erleichtert in einem vorgelagerten Schritt der eigentlichen Abscheidung von Flüssigkeit und Gas bereits in der Entgasungseinheit eine Auftrennung der beiden Medien. Das über den Hohlkanal des Resonators abgeleitete Flüssigkeits-/Gas-Gemisch kann über einen angelegten Überdruck in der Entgasungseinheit oder einem eingestellten Unterdruck in der Abscheidekammer, die außerhalb der Entgasungseinheit angeordnet ist, ausgeleitet werden. Über- und Unterdruck beziehen sich dabei auf den Normaldruck der Umgebungsatmosphäre.

Entsprechend dieser Anordnung von Resonator und Entgasungseinheit ist vorteilhaft weiter ein Entgasungsventil an der Entgasungkammer angebracht, über das bei auftretendem Überdruck bereits in der Entgasungseinheit abgeschiedenes Gas ausgeleitet werden kann. Zusätzlich ist in dieser Anordnung darauf zu achten, dass das Flüssigkeitsniveau nicht unter das Niveau der Resonatorspitze abfällt, da sonst Luft über den Hohlkanal des Resonators abgeleitet werden würde, was den Abscheideprozess in der nachfolgenden Abscheidekammer beeinträchtigen würde. Es kann daher vorzusehen sein, einen entsprechenden Sensor innerhalb oder außerhalb der Entgasungseinheit anzubringen, der ein Flüssigkeitsniveau feststellen kann. Über einen Kontroller, z. B. eine Prozessoreinheit, kann das Signal entsprechend bewertet werden. Für den Fall, dass das Flüssigkeitsniveau gegenüber dem Niveau der Resonatorspitze zu niedrig ist, wird der Entgasungsprozess oder die Ableitung durch den Hohlkanal entsprechend unterbrochen.

Eine weitere Ausführung sieht eine Anordnung einer Entgasungseinheit auf einem Einwegartikel vor, insbesondere einem Kassettenmodul zur Verarbeitung physiologischer Flüssigkeiten, z. B. in der Dialyse oder der Infusion. In dieser Anordnung ist vorgesehen, dass der Resonator ebenfalls in der Entgasungseinheit des Einwegartikels integriert ist, nicht jedoch der Schwinger. Der Resonator ist dabei so in der Entgasungseinheit angeordnet, dass er an einer Folie oder einem sonstigen flexiblen Wandungsabschnitt anliegt. Die Folie oder der flexible Wandungsabschnitt begrenzen und dichten die Entgasungseinheit zu einer Seite ab. Resonator, die anliegende Folie oder der flexible Wandungsabschnitt und Entgasungseinheit sind vorzugsweise aus Kunststoffmaterialien so gefertigt, dass die Nutzung des Kassettenmoduls als Einwegartikel wirtschaftlich möglich ist. Demzufolge sind insbesondere die schwingungsfesten Bauteile, wie die begrenzende Folie und der Resonator aus schwingungsfesten Materialien, insbesondere aus Materialien herzustellen, die schwingungsfest und kostengünstig sind. Unter schwingungsfest ist in diesem Zusammenhang zu verstehen, dass die Ultraschallleistung durch die schwingungsübertragenden Bauteile eine möglichst geringe Dämpfung erfährt.

Im Weiteren kann der Resonator wie in den Ausführungen zuvor beschrieben angeordnet sein. Je nach Konstruktionsbedingung des Kassettenmoduls oder der Art der zu entgasenden Flüssigkeit kann der Resonator ganz in das zu entgasende Flüssigkeitsvolumen eintauchen oder nur mit der Resonatorspitze in die Flüssigkeit eintauchen, solange die Anordnung gewährleisten kann, dass der Resonator über eine Fläche mit einer die Entgasungseinheit begrenzenden Folie oder einem flexiblen Wandungsabschnitt anliegen kann. In dieser Anordnung ist es möglich, den Schwinger separat von dem Einwegartikel bereitzustellen. Ein Entgasungsprozess findet dann statt, wenn das Kassettenmodul und die Entgasungseinheit mit dem Schwinger in schwingungsübertragenden Kontakt gebracht wird. Der Schwinger kann dabei in einer Konsole einer Maschine einer für die jeweilige Therapie des Patienten bereitgestellten Maschine bereitgestellt sein. Solche Maschinen stellen im Zusammenhang der vorliegend dargestellten Erfindung z. B. Dialysemaschinen oder Infusionsgeräte dar.

Technische Details zur Anordnungen von Maschinenkonsolen und Behandlungskassettenmodulen sind im Stand der Technik weitläufig beschrieben. Im vorliegenden Fall ist vorgesehen, dass der Konsolenteil einer Behandlungsmaschine zur Aufnahme eines Behandlungskassettenmoduls vorbereitet ist und dass der Schwinger in einem Teil der Konsole so angeordnet ist, dass er direkt benachbart zum Kassettenmodul liegen kann. Insbesondere ist die Geometrie von Kassette und Konsole so ausgestaltet, dass der Schwinger an der schwingungsfesten Folie oder dem schwingungsübertragenden, flexiblen Wandungsabschnitt der Entgasungseinheit anliegen kann, so dass die Schwingungen auf den Resonator und damit in das innere der Entgasungseinheit übertragen werden können. Diese Trennung von Schwinger und Resonator ermöglicht es in wirtschaftlich günstiger Weise, eine Ultraschall-Entgasung auf einem Einwegartikel zu verwirklichen.

Die Vorrichtung zur Entgasung von Flüssigkeiten ist weiterhin vorteilhaft so ausgebildet, dass sie einen Prozessor, einen Sensor und einen Regelkreislauf umfasst. Insbesondere wird durch diese Einrichtungen ein Regelungsmechanismus geschaffen, mit dem die Ultraschallleistung an veränderliche Umstände während der Entgasung angepasst werden kann. Insbesondere ist der Ultraschallgenerator und/oder der Schwinger mit einem Prozessor verbunden, der so konfiguriert ist, dass er über einen ermittelten Dämpfungswert einen Stellwert ermittelt und die Frequenz der Sonotrode so variiert, dass die Dämpfung minimal gehalten wird.

Am effizientesten wird der Ultraschallgenerator betrieben, wenn er in Resonanz mit dem umgebenden Flüssigkeitsmedium und der angrenzenden Wandung der Entgasungseinheit betrieben wird. Die akustischen Eigenschaften des Flüssigkeitsmediums können sich z. B. durch den Anteil an abgetrenntem Gas (z. B. Luftblasen in Wasser) oder einem veränderlichen Flüssigkeitspegel in der Entgasungseinheit verändern. Deshalb muss die Frequenz der Ultraschallsonotrode für den Betrieb (geringfügig) verstimmbar sein, um die "Resonanzfrequenz" immer erneut an veränderte Umgebungsparameter anzupassen, um im effizientesten Resonanzzustand zu arbeiten. Die Regelung kann durch eine im Stand der Technik bekannte Phasenregelschleife, z. B. auch genannt: "phase locked loop" (PLL), erfolgen.

Im Prinzip ist solch ein Schaltkreis eine "Mixed Signal"-Anwendung: Mit einem Schwellwert-Trigger wird der Zeitpunkt des ansteigenden Signalpegels (wird elektronisch gewonnen aus dem Strom, wichtig ist Phasenrichtigkeit) ermittelt. Zeitdifferenzen zum Sollwert werden in ein Frequenz veränderndes Signal umgesetzt. So wird eine Regelschleife gebaut, die eine ständige Anpassung erzeugt.

Die Erfindung betrifft weiterhin ein Verfahren zur Entgasung einer Flüssigkeit. Das Verfahren umfasst die Schritte des Einleitens einer Flüssigkeit in eine Entgasungseinheit, Eintauchen eines Resonators in die Flüssigkeit, Entgasen der Flüssigkeit durch Ultraschallbehandlung und Trennen der Flüssigkeit von darin gelöstem oder dispergierten Gasen und Ausleiten der Flüssigkeit und/oder des Gases aus der Entgasungseinheit.

Das Verfahren wird bevorzugt in der Entgasung von Flüssigkeiten angewendet, die als Behandlungsflüssigkeit in der extrakorporalen Blutbehandlung, insbesondere der Dialyse verwendet werden.

Für das Verfahren ist vorgesehen, dass der Ultraschallgenerator mit einer Leistung von 50 bis 1000 Watt betrieben wird. Bevorzugte Frequenzbereiche liegen zwischen 20 bis 40 kHz.

Das Flüssigkeitsmedium wird durch die eingetragenen Schallwellen von gelösten oder dispergierten Gaseinschlüssen getrennt. Die Trennung erfolgt über einen Schwinger an einem Resonator, der in die Flüssigkeit eintaucht.

Der Schwinger ist länglich, z. B. stabförmig ausgebildet, so dass eine effektive Trennung von Flüssigkeit und Gas an der Spitze des stabförmigen Schwingers, an dem die eingetragene Schallwellenleistung am größten ist, erfolgt. Bevorzugt ist diese Spitze mit hyperbolisch geformten Außenflächen aufgebaut. Durch die hyperbolische Formung können Kavitationsgeräusche in dem zu entgasenden Medium minimiert werden.

Das erfindungsgemäße Entgasungsverfahren ist weiterhin vorteilhaft dadurch gekennzeichnet, dass das an der Resonatorspitze erhaltene Flüssigkeits-/Gasgemisch (im folgenden Fluidgemisch) über einen Hohlkanal im Inneren des Schwingers abgeleitet wird. Das Fluidgemisch wird durch einen angelegten Überdruck in der Entgasungseinheit durch den Hohlkanal gefördert und in eine zweite Kammer abgeleitet, in der die Abscheidung von Gas und Flüssigkeit erfolgt. Äquivalent zu einer Überdruckanwendung in der Entgasungseinheit kann auch in der Abscheidekammer ein Unterdruck angelegt werden, so dass das Fluidgemisch aus der Entgasungseinheit in die Abscheidekammer gesaugt wird.

In der Abscheidekammer werden Gaskomponenten und Flüssigkeitskomponenten so von einander getrennt, dass die Flüssigkeitskomponente durch einen Ableitkanal als entgaste Flüssigkeit entnommen werden kann. Entsprechende Abscheideverfahren und -kammern sind im Stand der Technik, insbesondere an Dialysemaschinen, bereits gebräuchlich. Insbesondere wird hierbei unter Abscheidung verstanden, dass Flüssigkeit und Gasbestandteile sich zu zwei getrennten Phasen separieren. Damit wird das anfänglich in der Entgasungkammer enstehende disperse Gemisch aus Gas und Flüssigkeit vollständig in zwei Phasen ungewandelt.

Weiterhin kann die Flüssigkeit der Abscheidekammer entnommen werden und als Behandlungsflüssigkeit oder als eine Komponente einer Behandlungsflüssigkeit für die extrakorporale Blutbehandlung bereitgestellt werden.

### Kurze Beschreibung der Zeichnungen

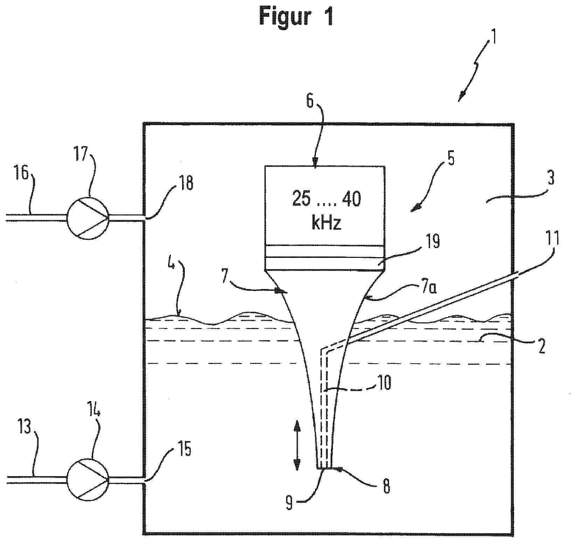

Figur 1 zeigt in schematischer Darstellung den Aufbau einer Entgasungseinheit für das Entgasen von medizinischen Behandlungsflüssigkeiten. Die Entgasungseinheit 1 umfasst ein Behältnis, in dem eine zur Entgasung bereitgestellte Flüssigkeit 2 lagert. Bei der Flüssigkeit kann es sich z. B. um eine Dialyselösung oder eine Komponente einer herzustellenden Dialyselösung handeln. Die Flüssigkeit kann kontinuierlich das Behältnis durchströmen, indem über die Öffnung 15, die Pumpe 14 und die Versorgungsleitung 13 kontinuierlich Flüssigkeit in das Behältnis nachgeführt wird. Das Behältnis ist nur teilweise mit der Flüssigkeit gefüllt. Über der Flüssigkeitsoberfläche 4 befindet sich ein Gasraum 3. Der Gasraum 3 kann üblicherweise mit Luft gefüllt sein, insbesondere mit steriler Luft, wenn medizinische Behandlungsflüssigkeiten entgast werden. Alternativ können auch weitere Gase, insbesondere Stickstoff oder Inertgase für die Entgasung bereitstehen. Die Gase können über eine Öffnung 18, eine Pumpe 17 über die Versorgungsleitung 17 der Entgasungseinheit zugeführt werden.

Die Entgasungseinheit 1 enthält weiterhin einen Ultraschalleinheit 5, die auch als Sonotrode zu bezeichnen ist, bestehend aus einem Schwinger 6 und einem Resonator 7. Der Resonator ist in dem vorliegenden Beispiel gemäß Figur 1 so aufgebaut, dass die Oberfläche 7a wenigstens teilweise eine hyperbolische Krümmung aufweist. In der länglichen hyperbolischen Ausgestaltung weist der Resonator eine Spitze 8 auf, die in das zu entgasende Flüssigkeitsmedium eintaucht. Im Inneren des Resonators befindet sich ein Hohlkanal 10, der in der Figur gestrichelt dargestellt ist und über die Öffnung 9 mit dem flüssigen Medium 2 in Kontakt steht. Über die Öffnung 9 und den Hohlkanal 10 kann ein ultraschallbehandeltes Flüssigkeits-/Gasgemisch aus der Entgasungseinheit 1 über die Öffnung 11 ausgeleitet werden und in einem weiteren, nicht dargestellten Gefäß zur Abscheidung von Flüssigkeit und Gas aufgefangen werden.

Während des Entgasungsvorgangs arbeitet der Ultraschallgenerator günstigstenfalls mit einer Frequenz von 25 bis 40 kHz. Über die Ankopplung 19 des Resonators wird dabei die mechanische Schwingungsenergie in das flüssige Medium 2 eingetragen. Der Resonator führt dabei im Wesentlichen Schwingungen in Richtung des in Figur 1 dargestellten Doppelpfeils aus. Da der Eintrag der mechanischen Energie an der Spitze 8 des Resonators am größten ist, findet hier die stärkste Entgasung direkt benachbart zur Öffnung 9 statt. Die Entgasung wird dadurch besonders effizient, weil das getrennte Flüssigkeits-/Gasgemisch direkt aus der Kammer ausgeleitet werden kann und eventuell Rücklösungsprozesse damit weitgehend vermieden werden können. Der Prozess des Ausleitens wird dabei durch Überdruckaufbau durch die Pumpen 14 oder 17 ermöglicht. Schematisch sind in Figur 1 beide Pumpen 14, 17 und die dazugehörenden Versorgungsleitungen 13, 16 eingezeichnet. Für den Betrieb sind jedoch nicht beide Pumpen und Versorgungsleitungen zwingend notwendig, so dass auch auf Pumpe 17 und Versorgungsleitung 16 verzichtet werden könnte. Pumpe 17 dient vor allem dazu einen angestrebten Überdruck in der Entgasungseinheit 1 zu erzeugen, indem der Entgasungseinheit ein Gas, wie z. B. Luft, zugeführt wird. Durch den eingestellten Überdruck kann die Rate reguliert werden, mit der an der Spitze 8 entgaste Flüssigkeit aus der Entgasungseinheit ausgeleitet wird.

In alternativen Ausführungen kann auf Pumpe 17, Versorgungskanal 16 und Öffnung 18 verzichtet werden. In solchen Ausführungen wird ein Überdruck durch die zugeführte Flüssigkeit über Pumpe 14 eingestellt und dadurch das Flüssigkeits-/Gasgemisch aus der Kammer 1 ausgeleitet. Das Gemisch wird in ein zweites Behältnis zur Abscheidung von Gas und Flüssigkeit übergeleitet, dass in Fig. 1 nicht dargestellt ist. Dem zweiten Behältnis, das auch als Abscheidekammer bezeichnet werden kann, wird die entgaste Flüssigkeit selektiv für die weitere Verwendung in der Dialysetherapie entnommen.

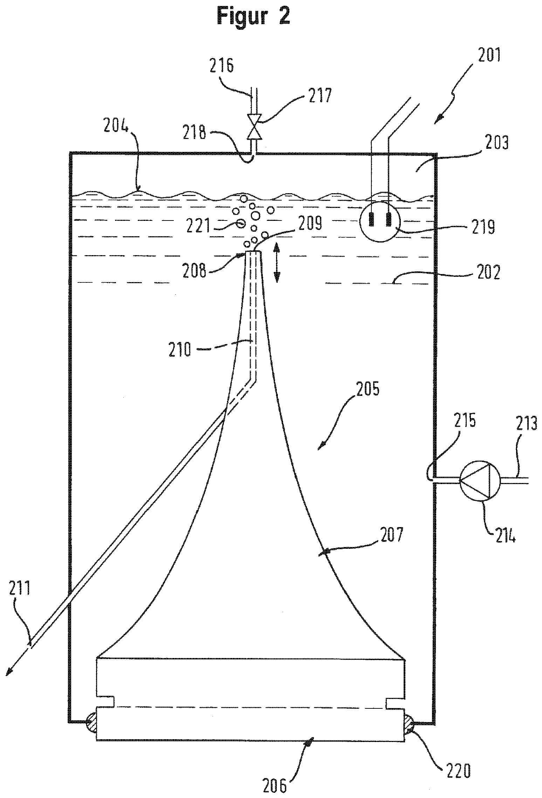

Figur 2 zeigt im Wesentlichen die Merkmale wie Figur 1, jedoch einer anderen Anordnung. Die Referenzzeichen entsprechen weitgehend denen aus Figur 2, jedoch sind zur besseren Zuordnung die Referenzzeichen nach Figur 2 fortlaufend von der Zahl 200 ausgehend nummeriert. Unterschiedlich zu Figur 2 ist die umgedrehte Anordnung von Resonator 207 und Schwinger 206 innerhalb der Entgasungseinheit 201. In der gezeigten Ausführung ist der Resonator 207 vollständig von der zu entgasenden Flüssigkeit 202 umgeben. Die Spitze 208 des Resonators 207 liegt benachbart zur Flüssigkeits-/Gas-Grenzfläche 204, aber innerhalb des Flüssigkeitsraumes 202.

Im Gegensatz zu der Ausführung nach Figur 1 ist der Schwinger 206 dichtend über die Dichtungen 220 in der Wandung der Entgasungseinheit 201 angeordnet. Durch einen Kanal 216 kann mittels eines Absperrventils 217 über die Öffnung 218 der Entgasungseinheit Gas aus der Entgasungseinheit ausgeleitet werden. Über eine Ansteuerung des Absperrventils kann so der Gasdruck über dem Flüssigkeitsvolumen reguliert werden und somit auch reguliert werden, mit welcher Flussrate das Gemisch aus entgaster Flüssigkeit und Gas durch den Ableitkanal 210, 211 aus der Entgasungseinheit ausgeleitet wird. Volumen und Druck des Gasraumes 203 werden durch sich bereits abscheidende Gasblasen 221 und durch den Kanal 213 zuströmende Flüssigkeit beeinflusst.

Beispielhaft ist ein Niveaufühler 219 gezeigt, mit dem geprüft werden kann, ob das Flüssigkeitsniveau für die Lage der Resonatorspitze 208 zu niedrig eingestellt ist oder nicht. Bei dem Niveaufühler kann es sich um einfache Elektroden handeln, mit deren Hilfe sich z. B. durch Messungen des elektrischen Widerstandes, der Impedanz oder der elektrischen Leitfähigkeit Aussagen über das Flüssigkeitsniveau machen lassen.

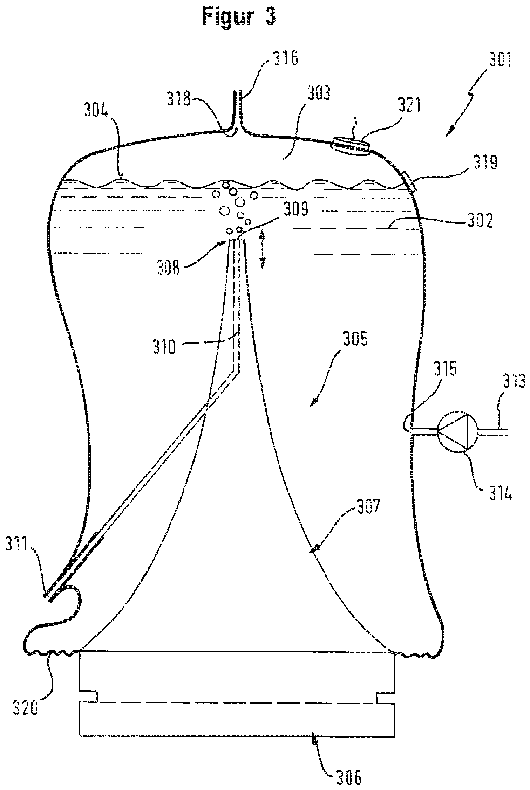

Figur 3 zeigt eine alternative Ausführung zu denen in Figuren 1 und 2 gezeigten Ausführungen. Darin wird die Anordnung aus einem externen Schwinger und einer Entgasungseinheit 301 mit Resonator gezeigt, wobei die Figur die Entgasungseinheit 301 in einem Ausschnitt eines Einwegartikels zeigt. Die Referenzzeichen stimmen weitgehend mit den Referenzzeichen der Figuren 1 und 2 überein, jedoch sind zur besseren Zuordnung die Referenzzeichen nach Figur 3 fortlaufend von der Zahl 300 nummeriert.

In Figur 3 ist der Resonator 307 innerhalb der Entgasungseinheit 301 eines Einwegartikels angeordnet. Dabei befindet sich der Resonator, wie in der Ausführung zu Figur 2, von zu entgasender Flüssigkeit umgeben. Die Spitze 308 des Resonators 307 befindet sich in Nachbarschaft zur Flüssigkeits-/Gas-Grenzfläche 304 innerhalb des Flüssigkeitsvolumens 302. Die Entgasungseinheit wird zu einer Seite durch die Membran 320, die dichtend mit der Entgasungseinheit verbunden ist, begrenzt. Bei der Membran handelt es sich um eine flexible Wandung, die auch als Folie ausgeführt sein kann. Vorteilhaft ist eine kautschukartige Membran, z. B. aus einer Mischung von Materialien, die aus der Gruppe der Materialien der Styrolblockcopolymeren, Polypropylenen, Poylethylenen, Poylalphaolefinen, deren Copolymeren und Blends hergestellt sein kann. Insbesondere ist ein Material zu wählen, das unter Schwingungsbedingungen des Schwingers schwingungsfest ist.

Angrenzend an die Membran 320 ist der Schwinger 306 angeordnet, der die Schwingungsenergie über die Membran 320 auf den Resonator 307 überträgt. Der Schwinger 306 ist extern zu dem Einmalartikel mit der Entgasungseinheit 301 angeordnet. Vorzugsweise ist der Schwinger in der Konsole einer Behandlungsmaschine angeordnet, die in Figur 3 nicht gezeigt ist.

Alternativ zu der Ausführung nach Figur 2 ist die Entgasungseinheit nach Figur 3 mit einem Levelsensor 319 ausgestattet, um das Flüssigkeitsniveau 304 bestimmen zu können. Der Sensor greift dabei nicht in die Flüssigkeit ein. Entsprechende Sensoren sind im Stand der Technik bekannt, z. B. magneto-resistive Sensoren oder Ultraschallsensoren.

An der Entgasungseinheit 301 nach Figur 3 kann weiterhin ein Drucksensor 321 angeordnet sein, der den Gasdruck des Gasraumes 303 messen kann. Über den Kanal 316 kann bei zu hohem Druck Gas aus der Kammer ausgeleitet werden. Alle zuleitenden und ableitenden Kanäle 318, 311, 313 können mit Ventilen versehen sein, die in der Figur nicht abgebildet sind, um weiterhin zuströmende und abströmende Fluide kontrollieren zu können.

Folgende Auflistung zeigt die in den Figuren 1, 2, und 3 verwendeten Referenzzeichen, deren Erläuterung und deren Übereinstimmungen:
Behältnis und Entgasungseinheit (1), (201), (301)
Flüssigkeitsraum/ zu entgasende Flüssigkeit (2), (202), (302)
Gasraum (3), (203), (303)
Flüssigkeits-/Gas-Grenzfläche (4), (204), (304)
Ultraschalleinheit (5), (205), (305)
Schwinger (6), (206), (306)
Resonator (7), (207), (307)
Resonatorspitze (8), (208), (308)
Öffnung in der Resonatorspitze (9), (209), (309)
Hohlkanal (10), (210), (310)
Öffnung zur Ableitung entgaster Flüssigkeit (11), (211), (311)
Flüssigkeitszuführkanal (13), (213), (313)
Zuführpumpe (14), (214), (314)
Zuführöffnung (15), (215), (315)
Zuführ-/ Abführkanal für Gas/ Luft (16), (216), (316)
Gaszuführ-/abführpumpe (17)
Gasventil (217)
Gaszuführ-/ abführöffnung (18), (218), (318)
Flüssigkeitsniveaufühler (219)
Levelsensor (319)
Gehäusedichtung (220)
Wandungsmembran (320)
abgeschiedene Gasblasen (221)
Drucksensor (321)

## Patentansprüche

1. Vorrichtung zum Entgasen einer Flüssigkeit für die Dialyse oder der extrakorporalen Blutbehandlung, umfassend ein Behältnis einer Entgasungseinheit (1) zur Aufnahme der Flüssigkeit (2) und eine Ultraschalleinheit (5) bestehend aus einer Ultraschallsonotrode mit einem Schwinger (6) und einem Resonator (7), wobei der Resonator (7) dafür geeignet ist, in die Flüssigkeit (2) des Behältnisses einzutauchen und eine Entgasung der Flüssigkeit durch Ultraschall zu bewirken, **dadurch gekennzeichnet, dass** der Resonator (7) einen Hohlkanal (10) beinhaltet, mittels dem die entgaste Flüssigkeit (2) aus dem Behältnis (1) abgeleitet werden kann.

2. Vorrichtung nach Anspruch 1, **dadurch gekennzeichnet, dass** der Resonator (7) eine längliche Ausdehnung aufweist, die als Spitze (8) ausgearbeitet ist.

3. Vorrichtung nach Anspruch 1 oder 2, **dadurch gekennzeichnet, dass** die Oberfläche (7a) des Resonators(7) eine hyperbolische Wölbung aufweist.

4. Vorrichtung nach einem oder mehreren der Ansprüche 1 bis 3, **dadurch gekennzeichnet, dass** die Entgasungseinheit mit Pumpmitteln (14, 17) verbunden ist, die in der Lage sind, einen Überdruck in dem Behältnis zu erzeugen.

5. Vorrichtung nach einem oder mehreren der Ansprüche 1 bis 4, **dadurch gekennzeichnet, dass** ein zweites Behältnis mit dem ableitenden Hohlkanal (10) des Resonators (7) verbunden ist, um die entgaste Flüssigkeit aufzunehmen.

6. Vorrichtung nach einem der Ansprüche 1 bis 5, **dadurch gekennzeichnet, dass** der Resonator (7) mit einem Schwinger (6) verbunden und als Schwimmer ausgebildet ist.

7. Vorrichtung nach einem oder mehreren der Ansprüche 1 bis 5, **dadurch gekennzeichnet, dass** die Entgasungseinheit so konstruiert ist, dass der Resonator (7) vollständig in die zu entgasende Flüssigkeit eintauchen kann.

8. Vorrichtung nach Anspruch 2, **dadurch gekennzeichnet, dass** die Resonatorspitze (8) unterhalb der Flüssigkeit/Gas-Grenzfläche (4) auf der Flüssigkeitsseite liegt.

9. Vorrichtung nach einem der Ansprüche 1 bis 8, **dadurch gekennzeichnet, dass** der Schwinger (6) oder der Resonator (7) mit einem Prozessor verbunden ist, der so konfiguriert ist, dass er über einen ermittelten Dämpfungswert einen Stellwert ermittelt und die Schwingungsfrequenz des Schwingers so variiert, so dass die Dämpfung minimal gehalten wird.

10. Anordnung aus einem Ultraschallschwinger und einem Einwegartikel mit einer Vorrichtung nach einem der Ansprüche 1 bis 9, wobei der Resonator (7) auf einem Einwegartikel und der Schwinger (6) separat vom Resonator (7) in der Konsole einer Behandlungsmaschine integriert sind und wobei der Einwegartikel und die Maschinenkonsole derart in Eingriff gebracht werden können, dass der Schwinger (6) schwingungsübertragend zum Resonator (7) am Einwegartikel angeordnet ist.

11. Einwegartikel mit einer Vorrichtung nach einem oder mehreren der Ansprüche 1 bis 9.

12. Verfahren zum Entgasen einer Flüssigkeit für die Dialyse oder extrakorporale Blutbehandlung umfassend die Schritte:
Einleiten einer Flüssigkeit (2) in ein Behältnis einer Entgasungseinheit (1), Eintauchen eines Resonators (7) in die zu entgasende Flüssigkeit, Entgasen der Flüssigkeit (2) durch Ultraschallbehandlung,
Ableiten von Flüssigkeit (2) oder abgetrenntem Gas durch einen Hohlkanal (10) des Resonators (7).

13. Verfahren nach Anspruch 12, **dadurch gekennzeichnet, dass** Flüssigkeit (2) und getrenntes Gas zusammen durch den Hohlkanal (10) abgeleitet und in einem zweiten Behältnis aufgefangen werden.

14. Verfahren nach Anspruch 13, **dadurch gekennzeichnet, dass** Flüssigkeit (2) und getrenntes Gas in dem zweiten Behältnis abgeschieden werden.

15. Verfahren nach Anspruch 13 oder 14, **dadurch gekennzeichnet, dass** die entgaste Flüssigkeit selektiv für eine weitere Verarbeitung dem zweiten Behältnis entnommen wird.

## Claims

1. Apparatus for degassing a liquid for dialysis or for an extracorporeal blood treatment, comprising a container of a degassing unit (1) for receiving the liquid (2) and an ultrasonic unit (5) consisting of an ultrasonic sonotrode with a transducer (6) and a resonator (7), the resonator (7) being suitable for immersion in the liquid (2) of the container and for inducing a degassing of the liquid by way of ultrasound, **characterized in that** the resonator (7) contains a hollow channel (10), by means of which the degassed liquid (2) can be removed from the container (1).

2. Apparatus according to Claim 1, **characterized in that** the resonator (7) has an elongate extent, which is formed into a tip (8).

3. Apparatus according to 1 or 2, **characterized in that** the surface (7a) of the resonator (7) has a hyperbolic curvature.

4. Apparatus according to one or more of Claims 1 to 3, **characterized in that** the degassing unit is connected to pumping means (14, 17) which are able to generate positive pressure in the container.

5. Apparatus according to one or more of Claims 1 to 4, **characterized in that** a second container is connected to the hollow removal channel (10) of the resonator (7) in order to receive the degassed liquid.

6. Apparatus according to any one of Claims 1 to 5, **characterized in that** the resonator (7) is connected to a transducer (6) and is embodied as a float.

7. Apparatus according to one or more of Claims 1 to 5, **characterized in that** the degassing unit is constructed in such a way that the resonator (7) can be completely immersed into the liquid to be degassed.

8. Apparatus according to Claim 2, **characterized in that** the resonator tip (8) is located below the liquid/gas interface (4) on the liquid side.

9. Apparatus according to any one of Claims 1 to 8, **characterized in that** the transducer (6) or the resonator (7) is connected to a processor which is configured such that it determines a control value by way of a determined damping value and varies the vibration frequency of the transducer such that the damping is kept to a minimum.

10. Arrangement of an ultrasonic transducer and a disposable article with an apparatus according to any one of Claims 1 to 9, wherein the resonator (7) is integrated in the disposable article and the transducer (6) is integrated separately from the resonator (7) in the console of a treatment machine and wherein the disposable article and the machine console can be made to engage in such a way that the transducer (6) is arranged at the disposable article so as to transfer vibrations to the resonator (7).

11. Disposable article comprising an apparatus according to one or more of Claims 1 to 9.

12. Method for degassing a liquid for dialysis or extracorporeal blood treatment, comprising the steps of:
introducing a liquid (2) into a container of a degassing unit (1),
immersing a resonator (7) into the liquid to be degassed, degassing the liquid (2) by ultrasonic treatment,
removing liquid (2) or separated gas through a hollow channel (10) of the resonator (7).

13. Method according to Claim 12, **characterized in that** liquid (2) and separated gas are removed together through the hollow channel (10) and captured in a second container.

14. Method according to Claim 13, **characterized in that** the liquid (2) and separated gas are separated out in the second container.

15. Method according to Claim 13 or 14, **characterized in that** the degassed liquid is selectively taken from the second container for further processing.

## Revendications

1. Dispositif de dégazage d'un liquide de dialyse ou pour un traitement extracorporel du sang, comprenant un récipient d'une unité de dégazage (1) pour recevoir le liquide (2) et une unité à ultrasons (5) constituée d'une sonotrode à ultrasons avec un oscillateur (6) et un résonateur (7), le résonateur (7) étant apte à plonger dans le liquide (2) du récipient et à provoquer un dégazage du liquide par ultrasons, **caractérisé en ce que** le résonateur (7) contient un conduit creux (10) au moyen duquel le liquide dégazé (2) peut être évacué hors du récipient (1).

2. Dispositif selon la revendication 1, **caractérisé en ce que** le résonateur (7) présente une étendue longitudinale qui est réalisée sous forme de pointe (8) .

3. Dispositif selon la revendication 1 ou 2, **caractérisé en ce que** la surface (7a) du résonateur (7) présente une courbure hyperbolique.

4. Dispositif selon l'une quelconque ou plusieurs des revendications 1 à 3, **caractérisé en ce que** l'unité de dégazage est connectée à des moyens de pompage (14, 17) qui sont en mesure de générer une surpression dans le récipient.

5. Dispositif selon l'une quelconque ou plusieurs des revendications 1 à 4, **caractérisé en ce qu'**un deuxième récipient est raccordé au conduit creux d'évacuation (10) du résonateur (7) afin de recevoir le liquide dégazé.

6. Dispositif selon l'une quelconque des revendications 1 à 5, **caractérisé en ce que** le résonateur (7) est raccordé à un oscillateur (6) et est réalisé sous forme de flotteur.

7. Dispositif selon l'une quelconque ou plusieurs des revendications 1 à 5, **caractérisé en ce que** l'unité de dégazage est construite de telle sorte que le résonateur (7) puisse plonger entièrement dans le liquide à dégazer.

8. Dispositif selon la revendication 2, **caractérisé en ce que** la pointe (8) du résonateur est située en dessous de la surface limite liquide/gaz (4) du côté liquide.

9. Dispositif selon l'une quelconque des revendications 1 à 8, **caractérisé en ce que** l'oscillateur (6) ou le résonateur (7) est raccordé à un processeur qui est configuré de manière à déterminer une valeur de réglage par le biais d'une valeur d'amortissement déterminée et de manière à faire varier la fréquence d'oscillation de l'oscillateur de telle sorte que l'amortissement soit maintenu minimal.

10. Agencement constitué d'un oscillateur à ultrasons et d'un article jetable comprenant un dispositif selon l'une quelconque des revendications 1 à 9, dans lequel le résonateur (7) sur un article jetable et l'oscillateur (6) séparément du résonateur (7) sont intégrés dans la console d'une machine de traitement et dans lequel l'article jetable et la console de machine peuvent être amenés en prise l'un avec l'autre de telle sorte que l'oscillateur (6) soit disposé sur l'article jetable avec transmission de l'oscillation au résonateur (7).

11. Article jetable comprenant un dispositif selon l'une quelconque ou plusieurs des revendications 1 à 9.

12. Procédé de dégazage d'un liquide de dialyse ou de traitement extracorporel du sang, comprenant les étapes suivantes :
introduction d'un liquide (2) dans un récipient d'une unité de dégazage (1),
immersion d'un résonateur (7) dans le liquide à dégazer, dégazage du liquide (2) par traitement aux ultrasons,
évacuation du liquide (2) ou du gaz séparé à travers un conduit creux (10) du résonateur (7).

13. Procédé selon la revendication 12, **caractérisé en ce que** le liquide (2) et le gaz séparé sont évacués ensemble à travers le conduit creux (10) et sont recueillis dans un deuxième récipient.

14. Procédé selon la revendication 13, **caractérisé en ce que** le liquide (2) et le gaz séparé sont séparés dans le deuxième récipient.

15. Procédé selon la revendication 13 ou 14, **caractérisé en ce que** le liquide dégazé est prélevé de manière sélective du deuxième récipient en vue d'un traitement ultérieur.
